# EUROPEAN PATENT SPECIFICATION

(11) **EP 2 789 357 B1**
(45) Date of publication and mention of the grant of the patent: **26.04.2017**
(21) Application number: 12854832.8
(22) Date of filing: 07.12.2012
(51) Int. Cl.: A61M 5/24, A61M 5/20

(54) **PHARMACEUTICAL SYRINGE UNIT AND PHARMACEUTICAL INJECTION DEVICE**
PHARMAZEUTISCHE SPRITZENEINHEIT UND PHARMAZEUTISCHE INJEKTIONSVORRICHTUNG
UNITÉ PHARMACEUTIQUE DE SERINGUE ET DISPOSITIF PHARMACEUTIQUE D'INJECTION

(30) Priority: 09.12.2011 JP 2011269692
(43) Date of publication of application: 15.10.2014
(73) Proprietor: Panasonic Healthcare Holdings Co., Ltd., Tokyo 105-8433 (JP)
(72) Inventor: KIKUCHI, Seiji, Osaka 540-6207 (JP); IIO, Toshiaki, Osaka 540-6207 (JP)
(74) Representative: Eisenführ Speiser
(86) International application number: PCT/JP2012/007846
(87) International publication number: WO 2013/084505

(56) References cited:
- EP-A1- 2 253 348
- WO-A1-2009/125582
- JP-A- 2003 245 350

## Description

### [Technological Field]

This disclosure relates to a pharmaceutical syringe unit containing a drug and a pharmaceutical injection device using the pharmaceutical syringe.

### [Background Technology]

Conventionally, a pharmaceutical syringe was formed into a cylindrical shape and provided with a mount for mounting an injection needle at its front end side, a piston-inserting part at its rear end side, and an indicator for indicating a type of a pharmaceutical syringe, such as, e.g., a bar code (See, for example, Patent Document 1).

Such a conventional bar code indicator of a pharmaceutical syringe is provided in the form of a belt along the outer circumference direction of the pharmaceutical syringe. This pharmaceutical syringe is mounted on a front end mounting portion of a pharmaceutical injection device and rotated to read the bar code indicator.

Also disclosed is a pharmaceutical injection device in which a color code provided on a pharmaceutical syringe is identified by a sensor provided on a syringe cover to detect the type of the drug (see, for example, Patent Document 2).

### [Prior Art Documents]

EP 2 253 348 A1 discloses a medication administering device that has mounted thereto a preparation syringe containing a preparation, that allows administration of a preparation to a living body, and that comprises a substantially cylindrical syringe cover that supports the preparation syringe on the inner peripheral face side, a piston that presses on the rear end of the preparation syringe, and a piston case that surrounds the piston, that guides the outer peripheral face of the syringe cover in the direction of the piston on the substantially cylindrical inner peripheral face side, and that is mounted in a state in which the syringe cover is removable.

### [Patent Document]

[Patent Document 1] Japanese Unexamined Laid-open Patent Application Publication No. H06-154322
[Patent Document 2] Japanese Unexamined Laid-open Patent Application Publication No. 2001-170176

### [Disclosure of the Invention]

### [Problems to Be Solved by the Invention]

However, in the case of the pharmaceutical injection device mentioned above, the bar code indicator cannot be appropriately read out unless the pharmaceutical syringe is appropriately positioned and mounted on the mount of the case body of the pharmaceutical injection device.

Further, although the pharmaceutical injection device mentioned above is required to position the color code on the pharmaceutical syringe and the sensor on the syringe cover, the positioning is difficult. Furthermore, since a sensor is provided on the syringe cover in this pharmaceutical injection device, it becomes complex to position a controller for processing data detected by the sensor and lay out its connection.

As a result, the code indicator cannot be read out appropriately, which prevents the drug injection.

The present invention aims to enable appropriate reading of a type of a drug.

### [Means for Solving the Problems]

A pharmaceutical syringe unit according to this disclosure is a pharmaceutical syringe unit to be mounted on a main body of a pharmaceutical injection device for injecting a drug filled therein into a body, the pharmaceutical syringe unit comprising a cylindrical syringe cover including at one end thereof a needle mount for an injection needle, a piston-inserting part at the other end, and a pharmaceutical syringe configured to be loaded in the syringe cover. The syringe cover or the pharmaceutical syringe includes, on an outer surface thereof, a discrimination mark that is detectable by the main body of the pharmaceutical injection device and indicates a type of the drug. The syringe cover includes, on an outer surface thereof, a rotation marker that indicates a rotation direction of the pharmaceutical syringe unit with respect to the main body of the pharmaceutical injection device by using an arrow when the pharmaceutical syringe unit is mounted on the main body.

The pharmaceutical injection device according to this disclosure comprises a pharmaceutical syringe unit, a main body including at one end side thereof a cylindrical syringe mount for mounting thereon the pharmaceutical syringe unit, and a detecting portion provided on the syringe mount of the main body and configured to detect a discrimination mark indicating a type of the drug, and a controller operable to determine a result detected by the detecting portion. The pharmaceutical syringe unit includes a cylindrical syringe cover including at one end a needle mount for an injection needle and a piston-inserting portion at the other end, and a pharmaceutical syringe configured to be loaded in the syringe cover. The pharmaceutical syringe unit further includes a discrimination mark that is detectable by the detecting portion and provided on an outer surface of the syringe cover or the pharmaceutical syringe. The discrimination mark and the detecting portion are arranged so as to be opposed to each other when the pharmaceutical syringe unit is mounted on the syringe mount.

### [Effects of the Invention]

The pharmaceutical syringe unit and the pharmaceutical injection device according to this disclosure make it possible to appropriately read a type of the drug.

### [Brief Description of the Drawings]

FIG. 1 is a schematic perspective view of a pharmaceutical injection device according to a first embodiment.
FIG. 2 is an exploded perspective view of the pharmaceutical injection device.
FIG. 3 is an enlarged perspective view showing a main section of the pharmaceutical injection device.
FIG. 4 is a cross-sectional view of the pharmaceutical syringe unit of the pharmaceutical injection device.
FIG. 5 is a cross-sectional view showing a main part of the pharmaceutical injection device.
FIG. 6 is a control block diagram of the pharmaceutical injection device.
FIG. 7 is a flow chart showing an operation of the pharmaceutical injection device.

### [Embodiment of the Invention]

Hereinafter, embodiments will be detailed with reference to the drawings. 1

### 1 First Embodiment

### 1-1 Configuration

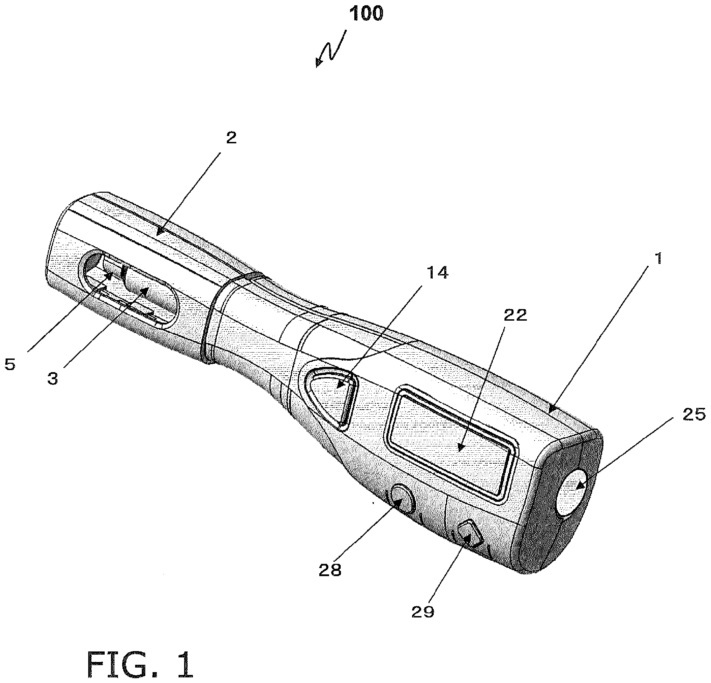

FIG. 1 is a perspective view showing an entirety of a pharmaceutical injection device 100 according to a first embodiment of the present invention.

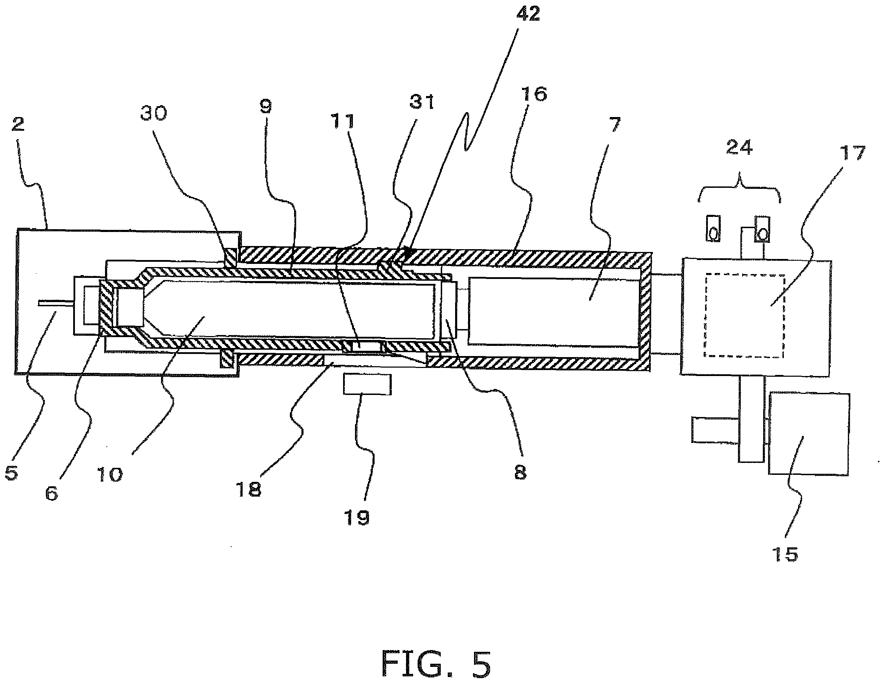

As shown in FIG. 1, the pharmaceutical injection device 100 (one example of a pharmaceutical injection device) is provided with a body case 1 (one example of a main body) and a front end cap 2 provided at a front end side of the body case 1. The pharmaceutical injection device 100 is further provided with an inner case 16 (one example of an inner case) arranged inside the body case 1 so as to be movable in a longitudinal direction of the body case 1 (see FIG. 5).

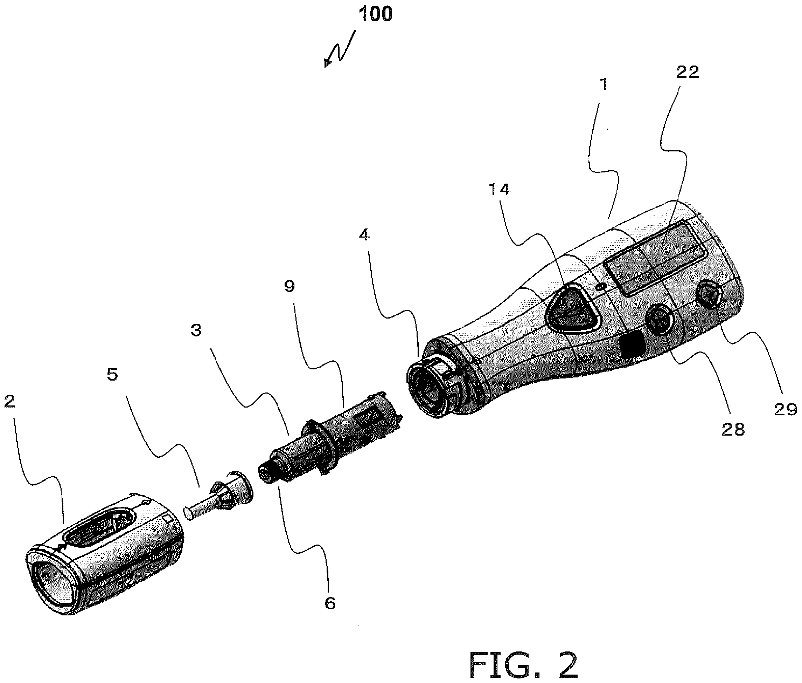

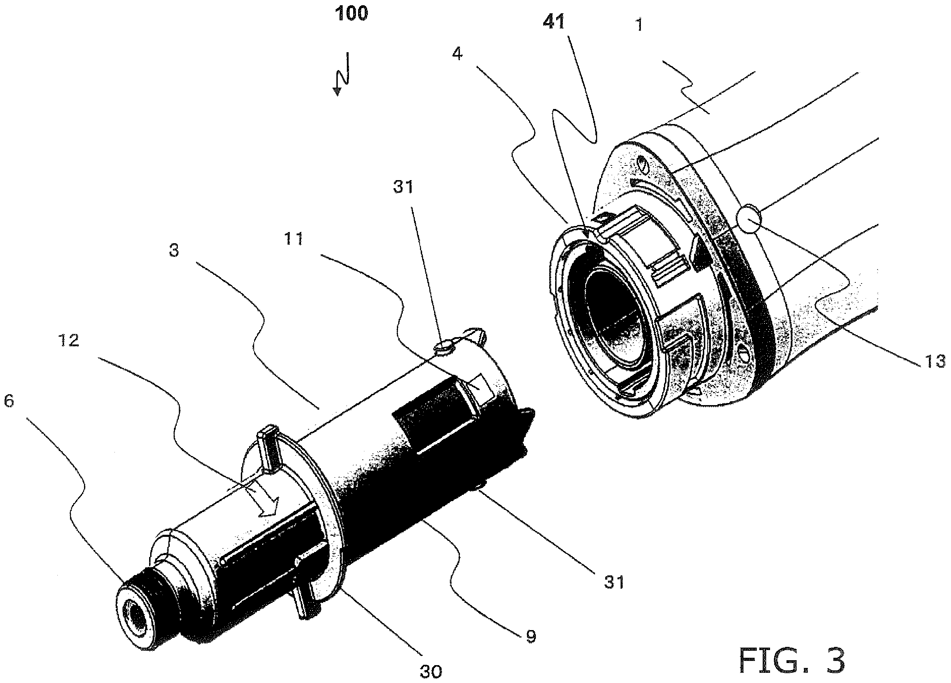

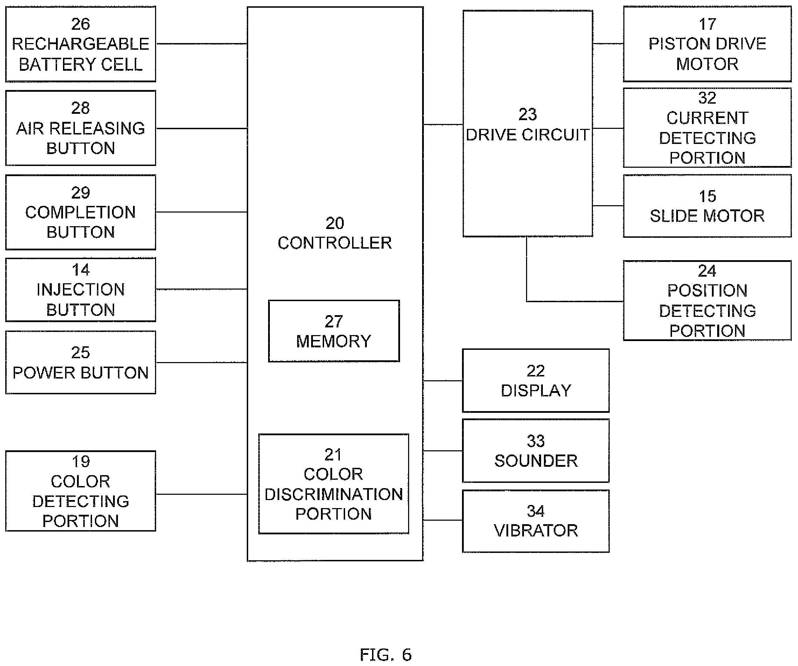

Further, as shown in FIGS. 2 and 3, the body case 1 has a syringe mount 4 (one example of a syringe mount) for mounting thereon a syringe unit 3 at a front end side of the body case 1. An insertion position marker 13 (one example of an indication portion) visually indicating the start position to insert the pharmaceutical syringe unit 3 is formed on the syringe mount 4, as will be detailed later. The body case 1 is further provided with a controller 20 (one example of a controller), various operation buttons, sensors and circuits connected to the controller 20 (see FIG. 6).

The inner case 16 is provided with an opening 18 on its side surface (see FIG. 5). When the pharmaceutical syringe unit 3 is mounted on the syringe mount 4 of the body case 1, the opening 18 is arranged so as to be opposed to a color detecting portion 19 (one example of a detecting portion) provided inside the body case 1. The color detecting portion 19 is constituted by, for example, an optical sensor or a color sensor.

Here, the color detecting portion 19, such as a color sensor, is configured to receive an input of color information (optical signals, etc.), convert the color information into an electrical signal and then output the electrical signal.

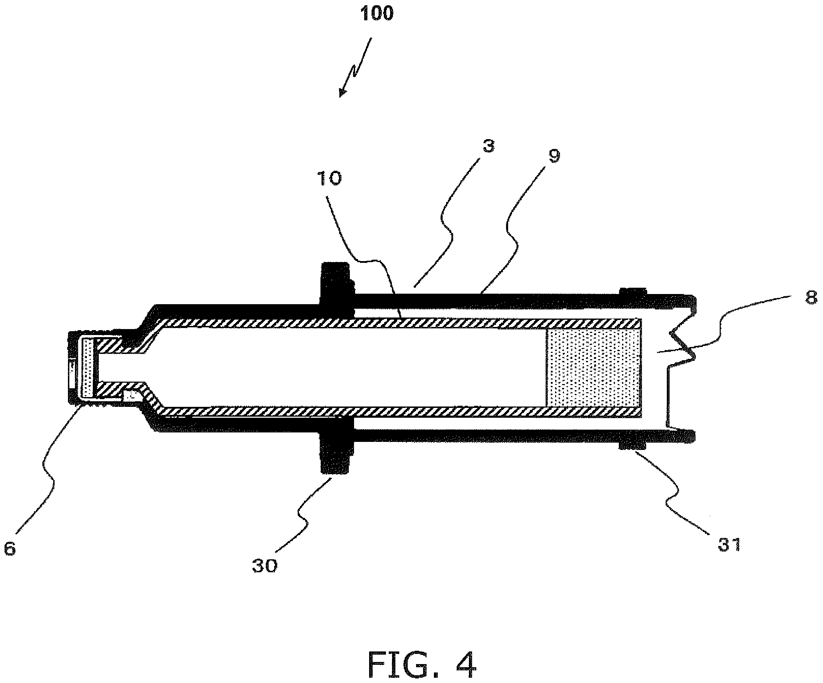

The pharmaceutical syringe unit 3 (one example of a pharmaceutical syringe unit) is, as shown in FIGS. 3 and 4, provided with a syringe cover 9 (one example of a syringe cover) and a pharmaceutical syringe 10 (one example of a pharmaceutical syringe) loaded in the syringe cover 9. The syringe cover 9 is cylindrical, and includes at a front end side a needle mount 6 for mounting thereon an injection needle 5 for injecting a drug into a body, and at the other end, an inserting part 8 into which a piston 7 for pushing a drug solution out of the pharmaceutical syringe is inserted as shown in FIG. 5. The injection needle 5 shown in FIG. 2 is an injection needle 5 in a state in which a needle cap is attached. Further, an islet-shaped colored mark 11 as one example of a discrimination mark indicating a type of the pharmaceutical syringe 10 for example is provided on the outer side surface of the rear part of the syringe cover 9, as shown in FIG. 3. The islet-shaped colored mark 11 is a visually recognizable mark capable of being detected by a color detecting portion 19 which will be described later. Further, the syringe cover 9 includes a rotation marker 12 indicating a rotation direction of the syringe cover 9 and located ahead of (on the needle mount 6 side of) the islet-shaped colored mark 11.

The islet-shaped colored mark 11 and the rotation marker 12 of the syringe cover 9 are arranged so as to be shifted in the outer circumferential direction of the syringe cover 9, i.e., in the rotation direction of the syringe cover 9.

Concretely, the rotation marker 12 is arranged so as to be shifted from the islet-shaped colored mark 11 rearward of the rotation direction of the syringe cover 9.

As shown in FIG. 3, at the time of mounting the pharmaceutical syringe unit 3 on the syringe mount 4 of the body case 1 of the pharmaceutical injection device 100, a user inserts the inserting part 8 located at the rear end of the syringe cover 9 into the cylindrical syringe mount 4 in a state in which the insertion position marker 13 of the syringe mount 4 and the islet-shaped colored mark 11 of the syringe cover 9 are in alignment with each other in the insertion direction (that is, the longitudinal direction of the body case 1). Next, the syringe cover 9 is rotated in the direction indicated by the rotation marker 12.

The pharmaceutical syringe unit 3 further includes, as shown in FIGS. 3 and 4, a flange 30 (one example of a flange portion) formed along the outer circumference of the cylindrical portion of the syringe cover 9 and protruded outward in a radial manner.

The flange 30 is formed such that it prevents invasion of the drug solution, etc., to the inside of the body case 1 from the front end side in a state in which the pharmaceutical syringe unit 3 is mounted on the syringe mount 4 of the body case 1. When the pharmaceutical syringe unit 3 is inserted into the syringe mount 4 of the body case 1, as shown in FIG. 5, the flange 30 engages a part of the body case 1 and stops the insertion.

Further, the pharmaceutical syringe unit 3 includes a removing and inserting protrusion 31 (one example of a protrusion) as shown in FIGS. 3 and 4. This removing and inserting protrusion 31 functions as a guide when the pharmaceutical syringe unit 3 is mounted on the syringe mount 4 of the body case 1. That is, the removing and inserting protrusion 31 is inserted in and along a concave shaped groove 41 (FIG. 3, one example of a first groove portion) formed inside the syringe mount 4 and on the inner side of the inner case 16. Thereafter, in accordance with the rotational movement of the pharmaceutical syringe unit 3, the removing and inserting protrusion 31 is engaged with and guided by a groove 42 (FIG. 5, one example of a second groove portion) communicated with the groove 41 and extended in the same direction as the direction indicated by the rotation marker 12. Thus, mounting of the pharmaceutical syringe unit 3 on the syringe mount 4 is completed.

FIG. 5 shows an inner cross-sectional view showing the state in which the pharmaceutical syringe unit 3 is mounted on the syringe mount 4 of the body case 1 (illustration of the body case 1, etc., is omitted). An injection needle 5 is mounted on the needle mount 6 at the front end of the syringe cover 9.

The injection needle 5 shown in FIG. 5 is in a state in which the needle cap is detached and a front end cap 2 is attached instead of the needle cap, so that the injection needle 5 is not exposed outside for safety.

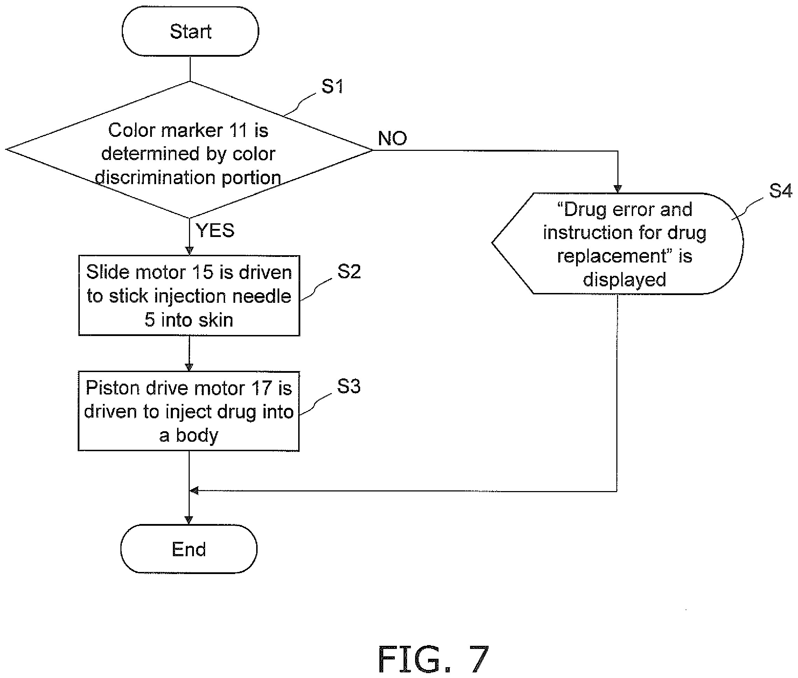

FIG. 6 is a control block diagram of the pharmaceutical injection device 100 according to the embodiment. The pharmaceutical injection device 100 includes a controller 20. The controller 20 is constituted by, e.g., a processor for executing a predetermined program to execute a function of each portion shown in FIG. 6, a memory 27 for storing the program and data to be processed by executing the program, etc. For example, the memory 27 stores programs for performing various controls (including the control flow shown in FIG. 7), set data for an amount of injection, injection history data, etc.

The controller 20 controls a drive circuit 23 to which a slide motor 15, a piston drive motor 17, a position detecting portion 24 and a current detecting portion 32 are connected. The controller 20 monitors the operation state of the drive circuit 23 and instruct and control the driving timing, etc., by the drive circuit 23. The drive circuit 23 drives the slide motor 15 and the piston drive motor 17 in accordance with the position detection by the position detecting portion 24.

The current detecting portion 32 detects current changes in the slide motor 15 and the piston drive motor 17 at the time of driving the motor or during the driving of the motor in each operation which will be explained later. The controller 20 monitors the abnormal operation of each motor by determining whether or not a sudden current change is detected by the current detecting portion 32. The controller 20 is also connected to a rechargeable battery cell 26, operation buttons (an air releasing button 28, a completion button 29, an injection button 14, a power button 25, etc.), a display 22, a sounder 33 and a vibrator 34. The controller 20 also includes a color discrimination portion 21 as a functional portion for discriminating the color of the islet-shaped colored mark 11 based on the detected result of the color detecting portion 19, which will be detailed later.

The power button 25 is a power button configured to turn on the power supply of the pharmaceutical injection device 100, and is configured to supply power to each portion from the rechargeable battery cell 26 when a user pushes the power button 25.

### 1-2 Operation

### 1-2-1 Drug Injection Operation

In the state shown in FIG. 5 (in the state in which the needle cap is removed from the injection needle 5 and a front end cap 2 is attached for safety. See FIG. 5), when the injection button 14 (see FIG. 1) of the pharmaceutical injection device 100 is pushed, the inner case 16 in the body case 1 moves forward (toward the needle mount 6 side) by the slide motor 15 shown in FIG. 5. In accordance with this movement, the entire pharmaceutical syringe unit 3 and the syringe cover 9 in the inner case 16 also move forward. As a result, the injection needle 5 mounted on the front end of the syringe cover 9 moves beyond the front end face of the front end cap 2 to be stuck into skin (hereinafter referred to as "needling operation").

Subsequently, the piston 7 is pushed forward by the piston drive motor 17, whereby the drug in the pharmaceutical syringe 10 will be injected into a human body through the injection needle 5 (hereinafter referred to as "injection operation").

When the injection of a predetermined amount of the drug is completed, the piston drive motor 17 is stopped. Further, as needed, after performing the operation for returning the piston 7 back to the original position, the piston drive motor 17 is stopped.

After the stop of the piston drive motor 17, the slide motor 15 is rotated reversely to pull out the injection needle 5 stuck into the skin and move it back to the inside of the front end cap 2 (hereinafter referred to as "needle pullout operation).

Further, normally, before the series of drug injection operations as explained above, an operation for releasing the air in the pharmaceutical syringe 10 to the outside (hereinafter referred to as "air releasing operation") is performed.

In this air releasing operation, the pharmaceutical injection device 100 releases the air in the pharmaceutical syringe 10 by performing basically similar operations as the above series of the drug injection operations with the injection needle 5 pointing not in a direction toward the skin but in the upward direction. As a matter of course, the travel distance of the piston 7 is set to a predetermined amount for air release, independently of the drug injection.

### 1-2-2 Discrimination Mark Detection Operation

Next, the detection operation of the islet-shaped colored mark 11 (see FIGS. 3 and 5) by the controller 20 will be explained with reference to FIGS. 6 and 7.

As explained above, the opening 18 is provided in the side surface of the inner case 16, and the color detecting portion 19 is positioned in the body case 1 so as to be opposed to the opening 18 (see FIG. 5).

In the drug injection operation as explained above, before the inner case 16 is moved forward together with the pharmaceutical syringe unit 3, the islet-shaped colored mark 11 of the syringe cover 9 passes the opening 18. At this time, the color of the islet-shaped colored mark 11 is optically read by the color detecting portion 19 via the opening 18 and converted into an electrical signal. The read electrical signal or information is output to the color discrimination portion 21 of the controller 20 shown in FIG. 6, and the type of the drug is determined at the color discrimination portion 21 (S1 in FIG. 7).

In particular, a signal indicating that color information of the islet-shaped colored mark 11 is electrically detected via the opening 18 by the color detecting portion 19 such as a color sensor is converted into an electrical signal and output to the color discrimination portion 21. The color discrimination portion 21 received an input of the electrical output signal from the color detecting portion 19, and determines whether or not an appropriate drug is loaded by detecting whether or not the output signal falls within a predetermined level (threshold) range with respect to the preliminarily set color.

As the simplest example, in a case where the appropriate color of the islet-shaped colored mark 11 of the syringe unit 3 is "blue," for example, "100-255" (when the resolving power is 256) is set as the "discrimination data for blue color" in the color discriminating set data region in the memory 27 in the controller 20 of the pharmaceutical injection device 100, and a blue-dedicated sensor that reacts only to the color "blue" is employed as the color sensor which is a color detecting portion 19. In this case, the color information of the islet-shaped colored mark 11 is input to the color detecting portion 19 constituted by the "blue-dedicated" sensor. When the islet-shaped colored mark 11 has an appropriate blue color, an electrical signal is output at a certain level or higher from the color detecting portion 19. Thereafter, the signal from the color detecting portion 19 (in this case, the signal corresponding to the blue color component) is input to the color discrimination portion 21, and the input signal (electrical signal) is A/D (analog/digital) converted to obtain a color information value, which is a digital value. Then, it is determined whether or not the obtained color value falls within the range of the above set threshold value as "blue-dedicated discrimination data" (in the above example, set to "100-255"), by which it is determined whether or not it is an appropriate pharmaceutical syringe. The discrimination result is forwarded to the controller 20.

Here, the reason that the "blue-dedicated discrimination data" is set to have the lower limit=100 is because if it is set to be excessively low, it would include a component of blue-black or a thin light blue with less blue component, and there is a possibility of being affected by the influence of external factors such as noise, it is set to a value of a certain level or above for accuracy. Of course, the aforementioned set value is one example, and may be set to an optimum value by arbitrarily changing the value depending on the color of the islet-shaped colored mark 11.

Further, as the color sensor, other than a single-color-dedicated color sensor such as the aforementioned blue-dedicated sensor, a color sensor that detects three colors of RGB can be used. Here, RGB denotes Red, Green, and Blue, which are called three primary colors of light, and the three primary colors of light are different from the three primary colors of a pigment system (paint, ink, etc.). In short, when paints of red, green and blue are superimposed, it becomes black (called "subtractive color mixing"). On the other hand, in the case of light, it becomes white (called "additive color mixing"). The three primary colors in the subtractive color mixing denotes yellow (Yellow), magenta (Magenta), and cyan (Cyan). In relation to the three primary colors of light, when R red and G green are superimposed, it makes yellow Y, when G green and B blue are superimposed, it makes cyan C, and then B blue and R red are superimposed, it makes magenta M. When R red, G green, and B blue are superimposed, it makes white.

As explained above, when an RGB sensor is used, the number of colors that can be identified increases as compared with the case of the aforementioned single-color-dedicated sensor. That is, in a case of using a RGB color sensor, which is a multicolor responsive color sensor, for example, yellow Y can be detected from the color information values of R red and G green, cyan C can be detected from the color information values of G green and B blue, magenta M can be detected from the color information values of R red and B blue, and other color information can be detected based on each color information value of each RGB. As a result, a color detection of multicolor can be easily performed. Accordingly, various information can be encoded and included in the discrimination mark of the syringe 9 as discrimination information on the syringe 9 or the drug in the syringe 9.

At the time of mounting the pharmaceutical syringe unit 3 on the syringe mount 4 of the pharmaceutical injection device 100, the rear end of the syringe cover 9 is inserted into the cylindrical syringe mount 4 in a state in which the insertion position marker 13 formed on the syringe mount 4 of the pharmaceutical injection device 100 and the islet-shaped colored mark 11 of the syringe cover 9 are aligned on a straight line. Next, the syringe cover 9 is rotated in the direction as indicated by the rotation marker 12 (see FIG. 3). This results in the state in which the islet-shaped colored mark 11 is arranged so as to be opposed to the opening 18 of the inner case 16 (see FIG. 5).

As explained above, before the drug injection operation, i.e., before the inner case 16 is moved forward, the color of the islet-shaped colored mark 11 of the syringe cover 9 is read by the color detecting portion 19 via the opening 18. Therefore, the type of the drug is appropriately determined by the color discrimination portion 21 shown in FIG. 6.

The color discrimination portion 21 connected to the controller 20 detects the islet-shaped colored mark 11 and transmits the result to the controller 20. The discrimination portion 21 of the controller 20 determines whether or not an appropriate pharmaceutical syringe 10 is mounted based on the information from the color detecting portion 19. When it is determined that an appropriate pharmaceutical syringe 10 is mounted, the slide motor 15 moves the inner case 16 in the body case 1 forward as mentioned above. Then, the drug injection operation is initiated. As a result of this, the injection needle 5 is stuck into the skin (S2 in FIG. 7). Subsequently, the piston 7 is pushed forward by the piston drive motor 17, whereby the drug in the pharmaceutical syringe 10 will be injected into a human body through the injection needle 5 (S3 in FIG. 7).

On the other hand, when the color discrimination portion 21 determines that an inappropriate pharmaceutical syringe 10 is mounted, the forward movement of the inner case 16 by the slider motor 15 or the sticking of the injection needle 5 into the skin, i.e., the drug injection to a human body, will not be performed.

When an inappropriate drug is mounted as mentioned above, a warning which indicates a drug error and a drug replacement instruction will be displayed on the display 22 of the body case 1 (S4 in FIG. 7).

In addition to the displaying of a warning display on the display 22, the controller 20 may also activate a sounder 33 (see FIG. 6) to give an alarm acoustically, or turn on the vibrator 34 (see FIG. 6) to give notice by vibrations.

### 1-3 Effects

As explained above, in this embodiment, the pharmaceutical syringe unit 3 includes the cylindrical syringe cover 9 having at one end side a needle mount 6 for the injection needle 5 , the piston-inserting part 8 at the other end side, and the pharmaceutical syringe 10 to be loaded in the syringe cover 9. The syringe cover 9 includes on the outer side surface the islet-shaped colored mark 11 that is detectable by the pharmaceutical injection device 100 and indicates a type of the drug. The syringe cover 9 includes on the outer side surface the rotation marker 12 that indicates the rotation direction of the pharmaceutical syringe unit with respect to the body case 1 when the pharmaceutical syringe unit 3 is mounted on the body case 1.

According to the configuration explained above, the islet-shaped colored mark 11 indicating a type of the drug and provided at the pharmaceutical syringe unit 3 is a mark when the pharmaceutical syringe unit 3 is mounted on the body case 1, and therefore the pharmaceutical syringe unit 3 can be easily and steadily mounted on the body case 1. Further, after the insertion of the pharmaceutical syringe unit 3 into the body case 1, the pharmaceutical syringe unit 3 is rotated in accordance with the rotation marker. Accordingly, the color detecting portion 19 provided at the body case 1 and the islet-shaped colored mark 11 can be arranged so as to be opposed to each other, and therefore, the type of the drug can be detected appropriately.

In this embodiment, the pharmaceutical injection device 100 is further provided with the pharmaceutical syringe unit 3, the body case 1 having at one end side the cylindrical syringe mount 4 for mounting thereon the pharmaceutical syringe unit 3, the color detecting portion 19 for detecting the islet-shaped colored mark 11 provided at the syringe mount 4 of the body case 1, and the controller 20 operable to determine the result detected by the color detecting portion 19. The pharmaceutical syringe unit 3 includes a cylindrical syringe cover 9 having at one end side a needle mount 6 for the injection needle 5 and the piston-inserting part 8 at the other end side, and the pharmaceutical syringe 10 to be loaded in the syringe cover 9. The pharmaceutical syringe unit 3 is further provided with the islet-shaped colored mark 11 on the outer side surface of the syringe cover 9. The islet-shaped colored mark 11 and the color detecting portion 19 can be arranged so as to be opposed to each other when the pharmaceutical syringe unit 3 is mounted on the syringe mount 4.

According to the configuration explained above, the islet-shaped colored mark 11 provided at the pharmaceutical syringe unit 3 can be detectable by the color detecting portion 19 provided at the main body side of the pharmaceutical injection device 100, and therefore it is not required to provide electrical connections and/or control functions to the pharmaceutical syringe unit 3, which is likely to be frequently attached/detached..

### 2 Other Embodiments

### 2-1

In the aforementioned embodiment, although the islet-shaped colored mark 11 is employed as the discrimination mark indicating the type of the pharmaceutical syringe 10, etc., the type or shape of the mark is not limited to it. As long as it takes a form in which a sensor reads information by the reflection of light, any mark of another type or shape can be employed. Further, in place of the color detecting portion 19, it is possible to provide a detecting portion depending on the type of the discrimination mark.

The position of the discrimination mark is not limited to the aforementioned embodiment, and can be, for example, at the center portion of the syringe cover 9 or at a position shifted forward thereof (on the needle mounting side).

Alternatively, the discrimination mark can be put on the pharmaceutical syringe 10. In this case, an opening is formed at a position on the side surface of the syringe cover 9 so as to be opposed to the discrimination mark. Further, the opening is provided at the position on the side surface of the inner case 16 so as to be opposed to the opening 18 when the pharmaceutical syringe unit 3 is mounted on the body case 1. With this, the discrimination mark provided on the pharmaceutical syringe 10 is read by the color detecting portion 19 via the opening on the syringe cover 9 and the opening 18 of the inner case 16.

Further, the aforementioned discrimination mark is not limited to one. A plurality of discrimination marks can be provided. In a case where the plurality of discrimination marks are color marks, the discrimination marks can be the same color or have different colors. In this case, a plurality of color detecting portions 19 are needed. The color discrimination portion 21 is not always required to be plural as long as it is provided with a circuit that switches terminals capable of inputting plural signals, or switches or processes plural signals.

### 2-2

When a user mounts the pharmaceutical syringe unit 3 to the body case 1, the controller 20 may display an explanation of how to mount it (e.g., operation guide function) on the display 22.

The controller 20 may be configured to display on the display 22 a notification notifying a start of using the pharmaceutical syringe or a supplemental explanation for a user to start its use when the discrimination mark such as the islet-shaped colored mark 11 is detected by a sensor and the information is discriminated.

### [Industrial applicability]

This disclosure is capable of being utilized as a pharmaceutical injection device used to cope with various illnesses or a pharmaceutical syringe unit for use in a pharmaceutical injection device.

### [Explanation of symbols]

- 1: Body case
- 2: Front end cap
- 3: Pharmaceutical syringe unit
- 4: Syringe mount
- 5: Injection needle
- 6: Needle mount
- 7: Piston
- 8: piston-inserting portion
- 9: Syringe cover
- 10: Pharmaceutical syringe
- 11: Islet-shaped colored marker
- 12: Rotation marker
- 13: Insertion position marker
- 14: Injection button
- 15: Slide motor
- 16: Inner case
- 17: Piston drive motor
- 18: Opening

## Claims

1. A pharmaceutical syringe unit (3) to be mounted on a main body of a pharmaceutical injection device (100) for injecting a drug filled therein into a body, comprising:
a cylindrical syringe cover (9) including at one end side thereof a needle mount (6) for an injection needle (5) and a piston-inserting portion (8) at the other end side; and
a pharmaceutical syringe (10) configured to be loaded in the syringe cover (9),
wherein the syringe cover (9) includes a flange portion (30) protruded outwardly from an outer surface of the syringe cover (9) in a radial manner,
the syringe cover (9) or the pharmaceutical syringe (10) includes, on an outer surface thereof, a discrimination mark that is detectable by the main body of the pharmaceutical injection device (100) and indicates a type of the drug,
the syringe cover (9) includes, on an outer surface thereof, a rotation marker (12) that indicates a rotation direction of the pharmaceutical syringe unit (3) with respect to the main body of the pharmaceutical injection device (100) by using an arrow when the pharmaceutical syringe unit (3) is mounted on the main body of the pharmaceutical injection device,
the rotation marker (12) is arranged at the one end side with respect to the flange portion (30), and
the discrimination mark is arranged at the other end side with respect to the flange portion (30).

2. The pharmaceutical syringe unit (3) according to claim 1, wherein the rotation marker (12) is arranged closer to the one end side than the discrimination mark is to the one end side.

3. The pharmaceutical syringe unit (3) according to claim 1 or 2, wherein the rotation marker (12) and the discrimination mark are arranged so as to be shifted in the rotation direction.

4. The pharmaceutical syringe unit (3) according to claim 2 or 3, wherein the rotation marker (12) is arranged so as to be rearward of the discrimination mark in the rotation direction.

5. A pharmaceutical injection device (100) comprising:
the pharmaceutical syringe unit (3) according to any of claims 1 to 4;
a main body including at one end side thereof a cylindrical syringe mount (4) for mounting thereon the pharmaceutical syringe unit (3);
a detecting portion (19) provided on the syringe mount (4) of the main body and configured to detect the discrimination mark;
a controller (20) operable to determine a result detected by the detecting portion (19); and
an inner case (16) configured to be slidable in the main body in a state where the inner case (16) accommodate the pharmaceutical syringe unit (3),
wherein the inner case (16) includes an opening (18) on a side surface thereof,
the opening (18) is arranged so as to be opposed to the discrimination mark and the detecting portion (19) when the pharmaceutical syringe unit (3) is mounted on the syringe mount (4) of the main body.

6. The pharmaceutical injection device (100) according to claim 5,
wherein the discrimination mark and the detecting portion (19) are arranged so as to be opposed to each other by a rotation of the pharmaceutical syringe unit (3) with respect to the main body when the pharmaceutical syringe unit (3) is mounted on the syringe mount (4).

7. The pharmaceutical injection device (100) according to claim 5 or 6,
wherein the main body includes an indication portion indicating (13) a mark indicating a start position to mount the pharmaceutical syringe unit (3), and
the detecting portion (19) is arranged so as to be shifted with respect to the indication portion (13) in a rotation direction of the pharmaceutical syringe unit (3) with respect to the main body.

8. The pharmaceutical injection device (100) according to claim 5,
wherein the pharmaceutical syringe unit (3) is configured to be mounted on the syringe mount (4) of the main body by being inserted therein,
the flange portion (30) is formed to stop an insertion of the syringe unit into the syringe mount (4) of the main body by being engaged with a part of the main body when the pharmaceutical syringe unit (3) is mounted on the syringe mount (4) of the main body, and
the rotation marker (12) is arranged so as to be exposed outside when the pharmaceutical syringe unit (3) is mounted on the syringe mount (4) of the main body.

9. The pharmaceutical injection device (100) according to any one of claims 5 to 8,
wherein the pharmaceutical syringe unit (3) includes a protrusion (31) for attaching and detaching the pharmaceutical syringe unit (3) to and from the syringe mount (4) of the main body, and
the syringe mount (4) of the main body includes a groove portion (41, 42) on an inner surface thereof, the groove portion (41, 42) being configured to receive the protrusion (31) and guide the protrusion (31).

10. The pharmaceutical injection device (100) according to claim 9,
wherein the groove portion (41, 42) includes a first groove portion (41) extending in a longitudinal direction of the pharmaceutical syringe unit (3) and a second groove portion (42) communicating with the first groove (41) and extending along an inner periphery of the syringe mount (4) of the main body.

11. The pharmaceutical injection device (100) according to claim 9 or 10, wherein the protrusion (31) is arranged so as to be shifted with respect to the discrimination mark in a rotation direction of the pharmaceutical syringe unit (3) with respect to the main body.

12. The pharmaceutical injection device (100) according to any one of claims 5 to 11,
wherein the main body further includes a display (22) portion that is controlled by the controller (20), and
the controller (20) controls the display (22) portion so as to display information indicating completion of mounting of the pharmaceutical syringe unit (3) to the syringe mount (4) of the main body when the discrimination mark is detected by the detection portion.

## Patentansprüche

1. Pharmazeutische Spritzeneinheit (3) zum Anbringen an einem Hauptkörper einer pharmazeutischen Injektionsvorrichtung (100) zum Injizieren eines darin eingefüllten Medikaments in einen Körper, umfassend:
eine zylindrische Spritzenabdeckung (9), die an ihrer einen Endseite eine Nadelaufnahme (6) für eine Injektionsnadel (5) und an der anderen Endseite einen Kolbeneinführabschnitt (8) aufweist; und
eine pharmazeutische Spritze (10), konfiguriert um in der Spritzenabdeckung (9) eingebracht zu sein,
wobei die Spritzenabdeckung (9) einen von einer Außenfläche der Spritzenabdeckung (9) radial nach außen vorstehenden Flanschabschnitt (30) aufweist,
die Spritzenabdeckung (9) oder die pharmazeutische Spritze (10) auf einer äußeren Oberfläche von dieser, eine Unterscheidungsmarkierung aufweist, die durch den Hauptkörper der pharmazeutischen Injektionsvorrichtung (100) erfassbar ist und eine Art des Arzneimittels anzeigt,
wobei die Spritzenabdeckung (9) auf einer äußeren Oberfläche von dieser eine Rotationsmarkierung (12) aufweist, die eine Drehrichtung der pharmazeutischen Spritzeneinheit (3) in Bezug auf den Hauptkörper der pharmazeutischen Injektionsvorrichtung (100) anzeigt, durch die Verwendung eines Pfeils, wenn die pharmazeutische Spritzeneinheit (3) an dem Hauptkörper der pharmazeutischen Injektionsvorrichtung angebracht ist,
die Rotationsmarkierung (12) an der einen Endseite in Bezug auf den Flanschabschnitt (30) angeordnet ist, und
die Unterscheidungsmarkierung an der anderen Endseite in Bezug auf den Flanschabschnitt (30) angeordnet ist.

2. Pharmazeutische Spritzeneinheit (3) nach Anspruch 1, wobei die Rotationsmarkierung (12) näher an der einen Endseite angeordnet ist, als die Unterscheidungsmarkierung an der einen Endseite angeordnet ist.

3. Pharmazeutische Spritzeneinheit (3) nach Anspruch 1 oder 2, wobei die Rotationsmarkierung (12) und die Unterscheidungsmarkierung so angeordnet sind, dass sie in Drehrichtung verschoben werden.

4. Pharmazeutische Spritzeneinheit (3) nach Anspruch 2 oder 3, wobei die Rotationsmarkierung (12) in der Drehrichtung hinter der Unterscheidungsmarkierung angeordnet ist.

5. Pharmazeutische Injektionsvorrichtung (100), umfassend:
die pharmazeutische Spritzeneinheit (3) nach einem der Ansprüche 1 bis 4;
einen Hauptkörper, der an seiner einen Endseite eine zylindrische Spritzenhalterung (4) aufweist, um darauf die pharmazeutische Spritzeneinheit (3) anzubringen;
einen Erfassungsabschnitt (19), der an der Spritzenhalterung (4) des Hauptkörpers vorgesehen ist und konfiguriert ist, um die Unterscheidungsmarkierung zu erfassen;
eine Steuereinrichtung (20), die betätigbar ist, um ein Ergebnis zu bestimmen, das durch den Erfassungsabschnitt (19) erfasst wird; und
ein Innengehäuse (16), das so konfiguriert ist, dass es in dem Hauptkörper in einem Zustand verschiebbar ist, in dem das Innengehäuse (16) die pharmazeutische Spritzeneinheit (3) aufnimmt,
wobei das Innengehäuse (16) eine Öffnung (18) an einer Seitenfläche von diesem aufweist,
wobei die Öffnung (18) so angeordnet ist, dass sie der Unterscheidungsmarkierung und dem Erfassungsabschnitt (19) gegenüberliegt, wenn die pharmazeutische Spritzeneinheit (3) an der Spritzenhalterung (4) des Hauptkörpers angebracht ist.

6. Pharmazeutische Injektionsvorrichtung (100) nach Anspruch 5,
wobei die Unterscheidungsmarkierung und der Erfassungsabschnitt (19) durch eine Drehung der pharmazeutischen Spritzeneinheit (3) in Bezug auf den Hauptkörper gegenüberliegend zueinander angeordnet sind, wenn die pharmazeutische Spritzeneinheit (3) an der Spritzenhalterung (4) angebracht ist.

7. Pharmazeutische Injektionsvorrichtung (100) nach Anspruch 5 oder 6,
wobei der Hauptkörper einen Indikatorabschnitt (13) aufweist, der eine Markierung anzeigt, die eine Anfangsposition zur Anbringung der pharmazeutischen Spritzeneinheit (3) anzeigt, und
der Erfassungsabschnitt (19) eingerichtet ist, um in Bezug auf den Indikatorabschnitt (13) in einer Drehrichtung der pharmazeutischen Spritzeneinheit (3) in Bezug auf den Hauptkörper verschoben zu werden.

8. Pharmazeutische Injektionsvorrichtung (100) nach Anspruch 5,
wobei die pharmazeutische Spritzeneinheit (3) so konfiguriert ist, dass sie an der Spritzenhalterung (4) des Hauptkörpers durch das Einsetzen in diesen angebracht wird,
der Flanschabschnitt (30) geformt ist, um ein Einführen der Spritzeneinheit in die Spritzenhalterung (4) des Hauptkörpers zu stoppen, indem er mit einem Teil des Hauptkörpers in Eingriff gebracht wird, wenn die pharmazeutische Spritzeneinheit (3) auf der Spritzenhalterung (4) des Hauptkörpers angebracht ist, und
die Rotationsmarkierung (12) so angeordnet ist, dass sie außen freiliegt, wenn die pharmazeutische Spritzeneinheit (3) an der Spritzenhalterung (4) des Hauptkörpers angebracht ist.

9. Pharmazeutische Injektionsvorrichtung (100) nach einem der Ansprüche 5 bis 8,
wobei die pharmazeutische Spritzeneinheit (3) einen Vorsprung (31) zum Anbringen und Abnehmen der pharmazeutischen Spritzeneinheit (3) an und von der Spritzenhaltung (4) des Hauptkörpers umfasst, und
die Spritzenhalterung (4) des Hauptkörpers einen Rillenabschnitt (41, 42) auf einer inneren Oberfläche von dieser einschließt, wobei der Rillenabschnitt (41, 42) dazu ausgebildet ist, den Vorsprung (31) aufzunehmen und den Vorsprung (31) zu führen.

10. Pharmazeutische Injektionsvorrichtung (100) nach Anspruch 9,
wobei der Rillenabschnitt (41, 42) einen ersten Rillenabschnitt (41) aufweist, der sich in einer Längsrichtung der pharmazeutischen Spritzeneinheit (3) erstreckt, und einen zweiten Rillenabschnitt (42) aufweist, der mit der ersten Rille (41) in Verbindung steht und sich entlang eines inneren Umfangs der Spritzenhalterung (4) des Hauptkörpers erstreckt.

11. Pharmazeutische Injektionsvorrichtung (100) nach Anspruch 9 oder 10, wobei der Vorsprung (31) so eingerichtet ist, um in Bezug auf die Unterscheidungsmarkierung in einer Drehrichtung der pharmazeutischen Spritzeneinheit (3) in Bezug auf den Hauptkörper verschoben zu werden.

12. Pharmazeutische Injektionsvorrichtung (100) nach einem der Ansprüche 5 bis 11,
wobei der Hauptkörper ferner einen Anzeigeabschnitt (22) umfasst, der durch die Steuereinrichtung (20) gesteuert wird, und
die Steuereinrichtung (20) den Anzeigeabschnitt (22) so steuert, um Informationen anzuzeigen, die den Abschluss der Montage der pharmazeutischen Spritzeneinheit (3) an der Spritzenhalterung (4) des Hauptkörpers erkennen lassen, wenn die Unterscheidungsmarkierung durch den Erfassungsabschnitt erfasst wird.

## Revendications

1. Unité de seringue pharmaceutique (3) destinée à être montée sur un corps principal d'un dispositif d'injection pharmaceutique (100) destiné à injecter un médicament y introduit dans un corps, comprenant:
une couverture cylindrique de seringue (9) qui, sur l'un de ses côtés d'extrémité, présente un logement d'aiguille (6) pour une aiguille d'injection (5) et, sur l'autre côté d'extrémité, présente une portion d'insertion de piston (8); et
une seringue pharmaceutique (10) configurée pour être chargée dans la couverture de seringue (9),
dans laquelle ladite couverture de seringue (9) comprend une portion de flasque (30) qui fait saillie radialement vers l'extérieur depuis une surface extérieure de la couverture de seringue (9),
la couverture de seringue (9) ou la seringue pharmaceutique (10) comprend, sur une surface extérieure de celle-ci, un repère discriminateur qui est détectable par le corps principal du dispositif d'injection pharmaceutique (100) et qui indique un type du médicament,
dans laquelle la couverture de seringue (9) présente, sur une surface extérieure de celle-ci, un repère de rotation (12) qui indique une direction de rotation de l'unité de seringue pharmaceutique (3) par rapport au corps principal du dispositif d'injection pharmaceutique (100) en utilisant une flèche, lorsque l'unité de seringue pharmaceutique (3) est montée sur le corps principal du dispositif d'injection pharmaceutique,
ledit repère de rotation (12) est agencé sur ledit un côté d'extrémité par rapport à la portion de flasque (30), et
ledit repère discriminateur est agencé sur l'autre côté d'extrémité par rapport à la portion de flasque (30).

2. Unité de seringue pharmaceutique (3) selon la revendication 1, dans laquelle le repère de rotation (12) est disposé plus près dudit un côté d'extrémité que le repère discriminateur l'est dudit un côté d'extrémité.

3. Unité de seringue pharmaceutique (3) selon la revendication 1 ou 2, dans laquelle le repère de rotation (12) et le repère discriminateur sont agencés de manière à être déplacés dans la direction de rotation.

4. Unité de seringue pharmaceutique (3) selon la revendication 2 ou 3, dans laquelle le repère de rotation (12) est agencé de manière à être en arrière du repère discriminateur dans la direction de rotation.

5. Dispositif d'injection pharmaceutique (100) comprenant:
ladite unité de seringue pharmaceutique (3) selon l'une quelconque des revendications 1 à 4;
un corps principal qui comprend, sur l'un de ses côtés d'extrémité, un support cylindrique de seringue (4) destiné à y monter ladite unité de seringue pharmaceutique (3);
une portion de détection (19) qui est prévue sur le support de seringue (4) du corps principal et est configurée pour détecter le repère discriminateur;
un dispositif de commande (19) apte à être opéré pour déterminer un résultat détecté par la portion de détection (19); et
un boîtier intérieur (16) configuré de manière à pouvoir coulisser à l'intérieur du corps principal dans un état où le boîtier intérieur (16) loge l'unité de seringue pharmaceutique (3),
dans lequel ledit boîtier intérieur (16) présente une ouverture (18) sur une surface latérale de celui-ci,
dans lequel ladite ouverture (18) est disposée de manière à être située en regard du repère discriminateur et de la portion de détection (19) lorsque l'unité de seringue pharmaceutique (3) est montée sur le support de seringue (4) du corps principal.

6. Dispositif d'injection pharmaceutique (100) selon la revendication 5,
dans lequel le repère discriminateur et la portion de détection (19) sont agencés de manière à être en regard l'un de l'autre par une rotation de l'unité de seringue pharmaceutique (3) par rapport au corps principal, lorsque l'unité de seringue pharmaceutique (3) est montée sur le support de seringue (4).

7. Dispositif d'injection pharmaceutique (100) selon la revendication 5 ou 6,
dans lequel le corps principal comprend une portion d'indication (13) indiquant un repère qui indique une position de départ pour le montage de l'unité de seringue pharmaceutique (3), et
la portion de détection (19) est agencée de manière à être déplacée par rapport à la portion d'indication (13) dans une direction de rotation de l'unité de seringue pharmaceutique (3) par rapport au corps principal.

8. Dispositif d'injection pharmaceutique (100) selon la revendication 5,
dans lequel l'unité de seringue pharmaceutique (3) est configurée pour être montée sur le support de seringue (4) du corps principal en y étant insérée,
la portion de flasque (30) est formée pour arrêter une insertion de l'unité de seringue dans le support de seringue (4) du corps principal en étant mise en prise avec une partie du corps principal lorsque l'unité de seringue pharmaceutique (3) est montée sur le support de seringue (4) du corps principal, et
le repère de rotation (12) est agencé de manière à être exposé à l'extérieur lorsque l'unité de seringue pharmaceutique (3) est montée sur le support de seringue (4) du corps principal.

9. Dispositif d'injection pharmaceutique (100) selon l'une quelconque des revendications 5 à 8,
dans lequel l'unité de seringue pharmaceutique (3) présente une projection (31) destinée à monter et détacher l'unité de seringue pharmaceutique (3) sur le et du support de seringue (4) du corps principal, et
le support de seringue (4) du corps principal comprend une portion de rainure (41, 42) sur une surface intérieure de celui-ci, ladite portion de rainure (41, 42) étant configurée pour recevoir la projection (31) et pour guider la projection (31).

10. Dispositif d'injection pharmaceutique (100) selon la revendication 9,
dans lequel la portion de rainure (41, 42) comprend une première portion de rainure (41) qui s'étend dans une direction longitudinale de l'unité de seringue pharmaceutique (3), et une deuxième portion de rainure (42) qui communique avec la première rainure (41) et s'étend le long d'une périphérie intérieure du support de seringue (4) du corps principal.

11. Dispositif d'injection pharmaceutique (100) selon la revendication 9 ou 10, dans lequel la projection (31) est agencée de manière à être déplacée par rapport au repère discriminateur dans une direction de rotation de l'unité de seringue pharmaceutique (3) par rapport au corps principal.

12. Dispositif d'injection pharmaceutique (100) selon l'une quelconque des revendications 5 à 11,
dans lequel le corps principal comprend en outre une portion d'affichage (22) qui est commandée par le dispositif de commande (20), et
le dispositif de commande (20) commande la portion d'affichage (22) de manière à afficher des informations indiquant l'achèvement du montage de l'unité de seringue pharmaceutique (3) sur le support de seringue (4) du corps principal, lorsque le repère discriminateur est détecté par la portion de détection.
